# EUROPÄISCHE PATENTANMELDUNG

(11) **EP 1 679 073 A1**
(43) Veröffentlichungstag der Anmeldung: **12.07.2006**
(21) Anmeldenummer: 06000157.5
(22) Anmeldetag: 05.06.2003
(51) Int. Cl.: A61K 31/4155, A61K 31/4178, A61K 31/433, A61K 31/42, C07D 403/12, C07D 417/12, C07D 413/14, C07D 403/14

(54) **2-(PHENYL)-2H-PYRAZOL-3-CARBONSÄURE-N-4-(IMINO-HETEROCYCLYL)-PHENYL-AMID DERIVATE SOWIE VERWANDTE VERBINDUNGEN ALS INHIBITOREN DER KOAGULATIONSFAKTOREN XA UND/ODER VIIA ZUR BEHANDLUNG VON THROMBOSENPhenylderivate 5**

(30) Priorität: 28.06.2002 DE 10229070
(62) Teilanmeldung aus: 03732540.4
(71) Anmelder: Merck Patent GmbH, 64293 Darmstadt (DE)
(72) Erfinder: Cezanne, Bertram, Dr., 64546 Mörfelden-Walldorf (DE); Dorsch, Dieter, Dr., 64372 Ober-Ramstadt (DE); Mederski, Werner, Dr., 64673 Zwingenberg (DE); Tsaklakidis, Christos, Dr., 69469 Weinheim (DE); Gleitz, Johannes, Dr., 64289 Darmstadt (DE); Barnes, Christopher, Dr., 65812 Bad Soden (DE)

(57) **Zusammenfassung**

Neue Verbindungen der Formel I 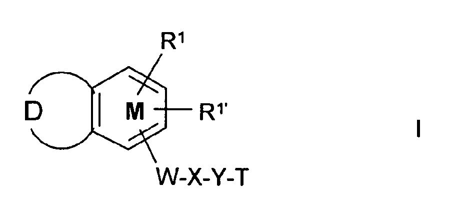 worin
D, M, W, X, Y, T, R¹ und R^{1'} die in Patentanspruch 1 angegebene Bedeutung haben,
sind Inhibitoren des Koagulationsfaktors Xa und können zur Prophylaxe und/oder Therapie von thromboembolischen Erkrankungen und zur Behandlung von Tumoren eingesetzt werden.

## Beschreibung

Die Erfindung betrifft Verbindungen der Formel I 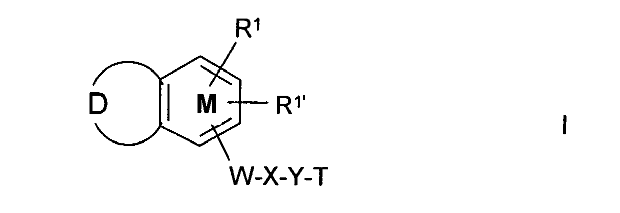 worin
- D: fehlt oder
eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch Hal, A,
-[C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²SO₂A, COR², SO₂NR² und/oder S(O)ₘA auftreten kann, und wobei ferner auch eine CH₂₋Gruppe der Alkylenkette durch eine C=O-Gruppe ersetzt sein kann,
- M: ein Phenylring oder ein aromatischer Heterocyclus, der 1-2 N- , O- und/oder S-Atome enthalten kann,
- R¹, R^{1'}: jeweils unabhängig voneinander H, Hal, A, OR², N(R²)₂, NO₂, CN, COOR² CON(R²)₂, C(=S)N(R²)₂, -[C(R³)₂]ₙ-Ar, -[C(R³)₂]ₙ-Het oder -[C(R³)₂]ₙ-Cycloalkyl, -[C(R³)₂]ₙ-N(R³)₂, CN, unsubstituiertes oder einfach durch C(=O)R³, COOR³, OR³, OCOR³, OCOOR³ oder durch eine konventionelle Aminoschutzgruppe substituiertes -C(=NH)-NH₂, 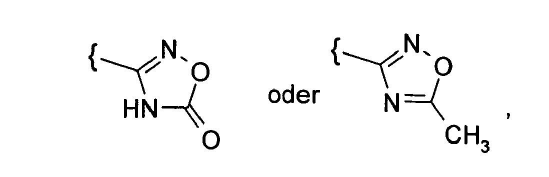
- R²: H, A, -[C(R³)₂]ₙ-Ar, -[C(R³)₂]ₙ-Het, -[C(R³)₂]ₙ-Cycloalkyl, -[C(R³)₂]ₙ-N(R³)₂ oder -[C(R³)₂]ₙ-OR³,
- R^{2'}: H, A, -[C(R³)₂]ₙ-Ar', -[C(R³)₂]ₙ-Het', -[C(R³)₂]ₙ-Cycloalkyl, -[C(R³)₂]ₙ-N(R³)₂ oder -[C(R³)₂]ₙ-OR³,
- R^{2"}: H, A, -[C(R³)₂]ₙ-Ar' oder -[C(R³)₂]ₙ-Cycloalkyl, -[C(R³)₂]ₙ-N(R³)₂ oder -[C(R³)₂]ₙ-OR³,
- R³: H oder A,
- W: einen ein- oder zweikernigen gesättigten, ungesättigten oder aromatischen Carbo- oder Heterocyclus mit 1 bis 4 N-, O- und/oder S-Atomen, der ein- oder zweifach durch R² substituiert sein kann,
- X: CONR², CONR²C(R³)₂, -C(R³)₂NR² oder -C(R³)₂NR²C(R³)₂, -C(R³)₂O-, -C(R³)₂OC(R³)₂- oder NR²CO,
- Y: Alkylen, Cycloalkylen, Het-diyl oder Ar-diyl,
- T: einen ein- oder zweikernigen gesättigten, ungesättigten oder aromatischen Carbo- oder Heterocyclus mit 1 bis 4 N-, O- und/oder S-Atomen, der ein- oder zweifach durch =S, =NR², =N-CN, =N-NO₂, =NOR², =NCOR², =NCOOR², =NOCOR² substituiert ist und ferner ein-, zwei- oder dreifach durch Hal, A, -[C(R³)₂]ₙ-Ar, -[C(R³)₂]ₙ-Het, -[C(R³)₂]ₙ-Cycloalkyl, OR³, N(R³)₂, NO₂, CN, COOR², CON(R²)₂, NR²COA, NR²CON(R²)₂, NR²SO₂A, COR², SO₂NR² und/oder S(O)ₘA substituiert sein kann,
- A: unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
- Ar: unsubstituiertes oder ein-, zwei- oder dreifach durch Hal, A, OR³, N(R³)₂, NO₂, CN, COOR³, CON(R³)₂, NR³COA, NR³CON(R³)₂, NR³SO₂A, COR³, SO₂N(R³)₂, S(O)ₘA, -[C(R³)₂]ₙ-COOR^{2'} oder -O-[C(R³)₂]ₒ-COOR^{2'} substituiertes Phenyl, Naphthyl oder Biphenyl,
- Ar': unsubstituiertes oder ein- oder zweifach durch Hal substituiertes Phenyl oder Benzyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =N(R³)₂, Hal, A, -[C(R³)₂]ₙ-Ar, -[C(R³)₂]ₙ-Het¹, -[C(R³)₂]ₙ-Cycloalkyl, -[C(R³)₂]ₙ-OR^{2'}, -[C(R³)₂]ₙ-N(R^{2'})₂, NO₂, CN, -[C(R³)₂]ₙ-COOR^{2'}, -[C(R³)₂]ₙ-CON(R^{2'})₂, -[C(R³)₂]ₙ-NR^{2'}COA, NR^{2'}CON(R^{2'})₂, -[C(R³)₂]ₙ-NR^{2'}SO₂A, COR^{2'}, SO₂NR^{2'} und/oder S(O)ₘA substituiert sein kann,
- Het¹: einen ein- oder zweikernigen gesättigten, ungesättigten oder aromatischen Heterocyclus mit 1 bis 2 N-, O- und/oder S-Atomen, der unsubstituiert oder ein- oder zweifach durch Carbonylsauerstoff, =S, =N(R³)₂, Hal, A, OR^{2"}, N(R^{2"})₂, NO₂, CN, COOR^{2"}, CON(R^{2"})₂, NR^{2"}COA, NR^{2"}CON(R^{2"})₂, NR^{2"}SO₂A, COR^{2"}, SO₂NR^{2"} und/oder S(O)ₘA substituiert sein kann,
- Hal: F, Cl, Br oder I,
- n: 0, 1 oder 2,
- m: 0, 1 oder 2,
- o: 1, 2 oder 3
bedeuten,
sowie ihre pharmazeutisch verwendbaren Derivate, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor Vlla, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben. Pyrazolderivate kennt man aus WO 01/29006 oder aus WO 02/24690.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor Vlla, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in *Circulation* **1996**, *94*, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in *Thrombosis and Haemostasis* **1990,** *63*, 220-223 beschrieben.

Die Messung der Inhibierung von Faktor Xa kann z.B. nach der Methode von T. Hara et al. in *Thromb. Haemostas.* **1994,** *71*, 314-319 erfolgen.

Der Gerinnungsfaktor Vlla initüert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors Vlla durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in *Thrombosis Research* **1996,** *84*, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in *Joumal of Biological Chemistry* **1998,** *273*, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor Vlla und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo*, oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-20 sowie ihrer pharmazeutisch verwendbaren Derivate, Solvate und Stereoisomere, dadurch gekennzeichnet, daß man
a) zur Herstellung einer Verbindung der Formel I,
   worin X CONR² oder CONR²C(R³)₂ bedeutet,
   eine Verbindung der Formel II 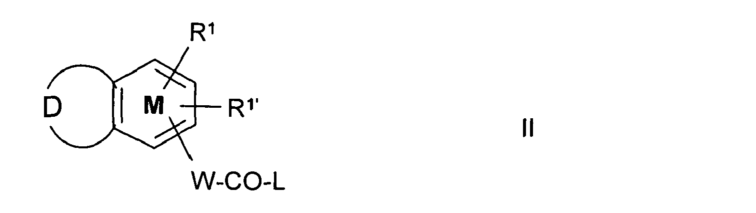 worin
   L Cl, Br, I oder eine freie oder reaktionsfähig funktionell abgewandelte OH-Gruppe bedeutet bedeutet
   und R¹, R^{1'}, D, M und W die in Anspruch 1 angegebenen Bedeutungen haben,
   mit der Maßgabe, daß falls eine weitere OH- und/oder Aminogruppe vorliegt, diese geschützt ist,
   mit einer Verbindung der Formel III

   Z'-Y-T III

   worin
   Z' NHR² oder NHR²C(R³)₂ bedeutet
   und R², Y und T die in Anspruch 1 angegebenen Bedeutungen haben,
   umsetzt,
   und anschließend gegebenenfalls eine Schutzgruppe abspaltet,
b) und/oder daß man in einer Verbindung der Formel I einen Rest T, R¹ und/oder R^{1'} in einen anderen Rest T, R¹ und/oder R¹ umwandelt,
indem man beispielsweise
i) eine Sulfanylverbindung in eine Iminoverbindung umwandelt,
ii) eine Aminoschutzgruppe abspaltet,
und/oder
eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter D, M, W, X, Y, T, R¹, R^{1'} die bei der Formel I angegebenen Bedeutungen, falls nicht ausdrücklich etwas anderes angegeben ist.

### Es bedeuten nachstehend:

- Ac: Acetyl
- BOC: tert.-Butoxycarbonyl
- CBZ oder Z: Benzyloxycarbonyl
- DCCI: Dicyclohexylcarbodiimid
- DCM: Dichlormethan
- DMF: Dimethylformamid
- EDCI: N-Ethyl-N,N'-(dimethylaminopropyl)-carbodiimid
- EE: Essigester
- Et: Ethyl
- Fmoc: 9-Fluorenylmethoxycarbonyl
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- HONSu: N-Hydroxysuccinimid
- OBut: tert.-Butylester
- Oct: Octanoyl
- OMe: Methylester
- OEt: Ethylester
- POA: Phenoxyacetyl
- TFA: Trifluoressigsäure
- Trt: Trityl (Triphenylmethyl).

Der Ring M bedeutet vorzugsweise Phenyl.

D kann, falls es vorliegt, ein- zwei- oder dreifach substituiert sein, vorzugsweise durch Hal, A, OR² oder N(R²)₂, auftreten kann, und/oder es kann auch eine CH₂-Gruppe der Alkylenkette durch eine C=O-Gruppe ersetzt sein. Ganz besonders bevorzugt ist eine einfach-Substitution durch A oder NH₂.

D bedeutet vorzugsweise -CO-NH-CO, -CO-NH-CH₂-, -NH-CH=CH-, -O-CH=CH-, -N=CH-O-, -N=CH-NH-, -NH-NH-CO-, -NH-N=N-, -NH-CO-CH₂-, -NH-CO-O-, -N=CH-S-, -NH-CO-S-, -NH-CO-NH-, -NH-N=CH-, -S-N=CH-, =C-S-N=, -O-N=CH-, -O-NH-CO-, -NH-O-CO-, -N=CH-CH=CH-, -CH=N-CH=CH-, -N=N-CH=CH-, -N=CH-N=CH-, -N=CH-CH=N-, -N=N-N=CH-, -NH-CO-CH=CH-, -NH-CH=CH-CO-, -NH-CO-CH₂-CH₂-, -NH-CH₂-CH₂-CO-, -NH-CO-N=CH-, -N=CH-NH-CO-, -NH-CO-NH-CO-, -NH-CO-NH-CH₂-, -CH=N-N=CH-, -N⁻-S⁺=-N-, -O-CH₂-O-, ferner -CH=N-NH-CO-, -CH=CH-NH-, -O-CH₂CH₂-O-, -CO-NH-NH-CO-, -N=N-NH-CO-, -O-CO-NH-CH₂- oder -O-CO-NH-CO-, wobei zusätzlich eine ein-, zwei- oder dreifache Substitution der Alkylenkette und/oder eines darin befindlichen Stickstoffes durch A oder NH₂, auftreten kann.

In einer weiteren Ausführungsform bedeutet D vorzugsweise eine gesättigte 3- bis 4-gliedrige Alkylenkette, bei der 1 bis 3 C-Atome durch N und/oder 1 bis 2 C-Atome durch 1 bis 2 O-Atome ersetzt sein können, wobei jedoch höchstens bis zu 3 C-Atome ersetzt werden und wobei zusätzlich eine ein- oder zweifache Substitution der Alkylenkette und/oder eines darin befindlichen Stickstoffes durch NH₂ auftreten kann, oder D fehlt.

D bedeutet besonders bevorzugt -CH=N-CH=CH-, -CH=CH-N=CH-, -NH-N=CH-, -CH=N-NH-, -O-N=CH- oder -CH=N-O-, wobei zusätzlich an D eine einfache Substitution durch NH₂ auftreten kann, oder D fehlt.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Alkoxy bedeutet vorzugsweise z.B. Methoxy, Ethoxy, Propoxy, Isopropoxy, Butoxy, Trifluormethoxy oder Cyclopentoxy.

Cycloalkyl bedeutet vorzugsweise Cyclopropyl, Cyclobutyl, Cylopentyl, Cyclohexyl oder Cycloheptyl.
Alkylen bedeutet vorzugsweise Methylen, Ethylen, Propylen, Butylen, Pentylen oder Hexylen, ferner verzweigtes Alkylen.

COR² bedeutet z.B. CHO oder -COA.
-COA (Acyl) bedeutet vorzugsweise Acetyl, Propionyl, ferner auch Butyryl, Pentanoyl, Hexanoyl oder z.B. Benzoyl.
Hal bedeutet vorzugsweise F, Cl oder Br, aber auch I.

R¹ bedeutet bevorzugt CN, CONH₂, CONA₂, NH₂, C(=S)NH₂, CH₂NH₂, CH₂CH₂NH₂, unsubstituiertes oder einfach durch OH, OCOA oder OCOOA substituiertes -C(=NH)-NH₂, 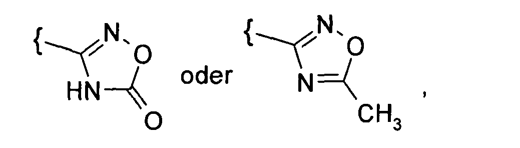 wobei A vorzugsweise Alkyl mit 1, 2, 3 oder 4 C-Atomen bedeutet.

R¹ bedeutet vorzugsweise H, -[C(R³)₂]ₙ-N(R³)₂, CON(R²)₂, C(=S)NH₂ oder N(R²)₂, besonders bevorzugt H, CH₂NH₂, NH₂, CONH₂ oder C(=S)NH₂.

R^{1'} bedeutet vorzugsweise H.

R² bedeutet vorzugsweise z.B. H oder Alkyl mit 1, 2, 3, 4, 5 oder 6 C-Atomen.
R^{2'} bedeutet vorzugsweise z.B. H oder Alkyl mit 1, 2, 3, 4, 5 oder 6 C-Atomen.
R^{2"} bedeutet vorzugsweise z.B. H oder Alkyl mit 1, 2, 3, 4, 5 oder 6 C-Atomen.

W bedeutet einen ein- oder zweikernigen gesättigten, ungesättigten oder aromatischen Carbo- oder Heterocyclus mit 1 bis 4 N-, O- und/oder S-Atomen, der ein- oder zweifach durch R² substituiert sein kann.

In einer bevorzugten Ausführungsform bedeutet W einen einkernigen gesättigten, ungesättigten oder aromatischen Carbo- oder Heterocyclus mit 1 bis 2 N-, O- und/oder S-Atomen, der ein- oder zweifach durch R² substituiert sein kann.

In einer weiteren bevorzugten Ausführungsform bedeutet W z.B. unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazol-diyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl. In einer weiteren bevorzugten Ausführungsform bedeutet W z.B. unsubstituiertes oder ein- oder zweifach durch A oder Hal substituiertes Cyclohexan-diyl, Cyclopentan-diyl, Phenylen, Biphenylen, Furan-diyl, Thiophen-diyl, Pyrrol-diyl, Imidazol-diyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl.

W bedeutet besonders bevorzugt unsubstituiertes oder einfach durch A, CONH₂ oder COOA substituiertes Pyrazol-diyl oder Thiazol-diyl, wobei A insbesondere Alkyl mit 1, 2, 3, 4, 5 oder 6 C-Atomen oder CF₃ bedeutet.

X bedeutet vorzugsweise CONH, CONHCH₂, CH₂NH oder CH₂NHCH₂, ganz besonders bevorzugt CONH.

Y bedeutet vorzugsweise Alkylen oder Ar-diyl, besonders bevorzugt Methylen, Ethylen, Propylen oder unsubstituiertes oder einfach durch A, Cl oder F substituiertes 1,4-Phenylen, ferner auch Pyridin-diyl, vorzugsweise Pyridin-2,5-diyl.
Y bedeutet insbesondere unsubstituiertes oder einfach durch Methyl, Ethyl, Propyl, Br, CI oder F substituiertes 1,3- oder 1,4-Phenylen, ganz besonders bevorzugt ist 1,4-Phenylen.

Ar bedeutet vorzugsweise unsubstituiertes oder ein-, zwei- oder dreifach durch Hal, A, OH, NH₂, NO₂, CN, COOH, CONH₂, NHCOA, NHCONH₂, NHSO₂A, COH, SO₂NH₂, S(O)ₘA, -(CH₂)ₙ-COOR^{2'} oder -O-(CH₂)ₒ-COOR^{2'} substituiertes Phenyl, Naphthyl oder Biphenyl.

Ar bedeutet z.B. unsubstituiertes Phenyl, Naphthyl oder Biphenyl, weiterhin vorzugsweise z.B. durch A, Fluor, Chlor, Brom, lod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Het bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het¹ hat die bevorzugten Bedeutungen wie Het.

T bedeutet vorzugsweise einen einkernigen gesättigten oder ungesättigten Heterocyclus mit 1 bis 2 N- und/oder O- Atomen, der ein- oder zweifach durch =S, =NR², =NOR², =N-CN, =N-NO₂, =NCOR² =NCOOR², =NOCOR² substituiert ist, insbesondere durch =S, =NR² oder =NOR², sowie vorzugsweise weiterhin ein- oder zweifach durch A substituiert sein kann.

T bedeutet insbesondere ein- oder zweifach durch =NR², =S oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H*-Pyridazin-2-yl, Azepan-1-yl, 2-Aza-bicyclo[2.2.2]-octan-2-yl, Pyrazol-2-yl, [1,3,4]-Thiadiazol-3-yl, lmidazolidin-1-yl oder 1,2-Dihydropyrazol-2-yl, die vorzugsweise weiterhin ein- oder zweifach durch A, CONH₂ oder COOA substituiert sein können.

T bedeutet weiterhin besonders bevorzugt z.B. 2-Imino-piperidin-1-yl, 2-Imino-pyrrolidin-1-yl, 2-Imino-1*H*-pyridin-1-yl, 3-Imino-morpholin-4-yl, 4-Imino-1*H*-pyridin-1-yl, 2,6-Diimino-piperidin1-yl, 2-Imino-piperazin-1-yl, 2,6-Diimino-piperazin-1-yl, 2,5-Diimino-pyrrolidin-1-yl, 2-Imino-1,3-oxazolidin-3-yl, 3-Imino-2*H*-pyridazin-2-yl, 2-Imino-azepan-1-yl, 2-Hydroxy-6-imino-piperazin-1-yl, Pyrazol-2-yl, 1,2-Dihydropyrazol-2-yl, 2-Methoxy-6-imino-piperazin-1-yl, 2-Imino-[1,3,4]-thiadiazol-3-yl, 2-Imino-imidazolidin-1-yl ganz besonders bevorzugt ist 2-Imino-piperidin-1-yl, sowie die entsprechenden Hydroxyimino-, Alkoxyimino-, Thioxo- und =N-(CH₂)₁₋₃NA'₂ - Derivate, wobei A' Alkyl mit 1, 2, 3, 4, 5, oder 6 C-Atomen bedeutet.

T bedeutet in einer weiteren besonders bevorzugten Ausführungsform z.B. 2-Imino-pyrrolidin-1-yl, 2-Imino-piperidin-1-yl, 2-Imino-[1,3,4]thiadiazol-3-yl, 2-Imino-imidazolidin-1-yl oder 3-Imino-1,2-Dihydro-pyrazol-2-yl, sowie die entsprechenden Hydroxyimino-, Cyanimino-, Alkoxyimino- und Thioxo-Derivate, wobei die heterocyclischen Reste vorzugsweise weiterhin ein- oder zweifach durch A, CONH₂ oder COOA substituiert sein können.

T bedeutet in einer weiteren besonders bevorzugten Ausführungsform z.B. 2-Imino-pyrrolidin-1-yl, 2-Imino-piperidin-1-yl, 2-Thioxo-pyrrolidin-1-yl, 2-Imino-[1,3,4]thiadiazol-3-yl, 2-Methoxy-imino-pyrrolidin-1-yl, 2-Hydroxyimino-pyrrolidin-1-yl, 2-Imino-imidazolidin-1-yl oder 3-Imino-1,2-Dihydro-pyrazol-2-yl, wobei die heterocyclischen Reste vorzugsweise weiterhin ein- oder zweifach durch A, CONH₂ oder COOA substituiert sein können.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Iw ausgedrückt werden, die der Formel I entsprechen und worin die nicht näher bezeichneten Reste die bei der Formel I angegebene Bedeutung haben, worin jedoch
- in la: D fehlt
bedeutet;
- in lb: M ein Phenylring
bedeutet;
- in lc: in Ic
D eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch Hal, A, OR² oder N(R²)₂, auftreten kann, und wobei ferner auch eine CH₂-Gruppe der Alkylenkette durch eine C=O-Gruppe ersetzt sein kann,
bedeutet;
- in ld: D eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch A oder NH₂, auftreten kann,
bedeutet;
- in le: D fehlt oder eine gesättigte 3- bis 4-gliedrige Alkylenkette, bei der 1 bis 3 C-Atome durch N und/oder 1 bis 2 C-Atome durch 1 bis 2 O-Atome ersetzt sein können, wobei jedoch höchstens bis zu 3 C-Atome ersetzt werden
bedeutet,
und wobei zusätzlich eine ein- oder zweifache Substitution der Alkylenkette und/oder eines darin befindlichen Stickstoffes durch NH₂ auftreten kann,
- in lf: D fehlt oder -CH=N-CH=CH-, -CH=CH-N=CH-, -NH-N=CH-, -CH=N-NH-, -O-N=CH- oder -CH=N-O-,
bedeutet,
und wobei zusätzlich an D eine einfache Substitution durch NH₂ auftreten kann;
- in lg: R¹ H, -[C(R³)₂]ₙ-N(R³)₂, CON(R²)₂, C(=S)NH₂ oder N(R²)₂, R^{1'} H,
bedeuten;
- in lh: R¹ H, CH₂NH₂, CONH₂, C(=S)NH₂ oder NH₂, R^{1'} H,
bedeuten;
- in li: W einen einkernigen gesättigten, ungesättigten oder aromatischen Carbo- oder Heterocyclus mit 1 bis 2 N-, O- und/oder S-Atomen, der ein- oder zweifach durch R² substituiert sein kann,
bedeutet;
- in lj: W unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazoldiyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl
bedeutet;
- in lk: W unsubstituiertes oder einfach durch A substituiertes Pyrazol-diyl,
bedeutet;
- in ll: X CONH, CONHCH₂, CH₂NH oder CH₂NHCH₂
bedeutet;
- in lm: X CONH bedeutet;
- in ln: Y Alkylen oder Ar-diyl bedeutet;
- in lo: Y unsubstituiertes oder ein- oder zweifach durch A, Br, Cl oder F substituiertes Phenylen
bedeutet;
- in lp: T einen einkernigen gesättigten oder ungesättigten Heterocyclus mit 1 bis 3 N- , O- und/oder S- Atomen, der ein- oder zweifach durch =S, =NR², =NOR², =N-CN, =N-NO₂, =NCOR², =NCOOR², =NOCOR² substituiert ist, sowie ein- oder zweifach durch A, CON(R²)₂ oder COOR² substituiert sein kann,
bedeutet;
- in lq: T einen einkernigen gesättigten oder ungesättigten Heterocyclus mit 1 bis 3 N- , O- und/oder S- Atomen, der ein- oder zweifach durch =S, =NR², =N-CN oder =NOR² substituiert ist, sowie ein- oder zweifach durch A, CON(R²)₂ oder COOR² substituiert sein kann,
bedeutet;
- in lr: T ein- oder zweifach durch =NR², =S, =N-CN, oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2- yl, Pyrazol-2-yl, Imidazolidin-1-yl, [1,3,4]-Thiadiazol-3-yl- oder 1,2-Dihydropyrazol-2-yl, die weiterhin ein- oder zweifach durch A, CONH₂ oder COOA substituiert sein können,
bedeutet;
- in ls: T 2-Imino-piperidin-1-yl, 2-Imino-pyrrolidin-1-yl, 2-Imino-1*H-*pyridin-1-yl, 3-Imino-morpholin-4-yl, 4-Imino-1*H*-pyridin-1-yl, 2,6-Diimino-piperidin1-yl, 2-Imino-piperazin-1-yl, 2,6-Diimino-piperazin-1-yl, 2,5-Diimino-pyrrolidin-1-yl, 2-Imino-1,3-oxazolidin-3-yl, 3-Imino-2*H*-pyridazin-2-yl, 2-Imino-azepan-1-yl, 2-Hydroxy-6-imino-piperazin-1-yl, Pyrazol-2-yl, 1,2-Dihydropyrazol-2-yl, 2-Methoxy-6-imino-piperazin-1-yl, 2-Imino-[1,3,4]-thiadiazol-3-yl, 2-Imino-imidazolidin-1-yl sowie die entsprechenden Hydroxyimino-, Alkoxyimino-, Thioxo- und =N-(CH₂)₁₋₃NA'₂ - Derivate, wobei A' Alkyl mit 1, 2, 3, 4, 5, oder 6 C-Atomen bedeutet, und wobei die Heterocyclen weiterhin ein- oder zweifach durch A, CONH₂ oder COOA substituiert sein können,
bedeutet;
- in lt: T T 2-Imino-pyrrolidin-1-yl, 2-Imino-piperidin-1-yl, 2-Imino-[1,3,4]thiadiazol-3-yl, 2-Imino-imidazolidin-1-yl oder 3-Imino-1,2-Dihydro-pyrazol-2-yl, sowie die entsprechenden Hydroxyimino-, Alkoxyimino- und Thioxo-Derivate, wobei die heterocyclischen Reste weiterhin ein- oder zweifach durch A, CONH₂ oder COOA substituiert sein können,
bedeutet;
- in lu: D D fehlt oder -CH=N-CH=CH-, -CH=CH-N=CH-, -NH-N=CH-, -CH=N-NH-, -O-N=CH- oder -CH=N-O-,,
M ein Phenylring,
R¹ H, CH₂NH₂, CONH₂, C(=S)NH₂ oder NH₂,
R^{1'} H,
W einen einkernigen gesättigten, ungesättigten oder aromatischen Carbo- oder Heterocyclus mit 1 bis 2 N-, O- und/oder S-Atomen, der ein- oder zweifach durch R² substituiert sein kann,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y Alkylen oder Ar-diyl,
Ar unsubstituiertes oder ein-, zwei- oder dreifach durch Hal, A, OH, NH₂, NO₂, CN, COOH, CONH₂, NHCOA, NHCONH₂, NHSO₂A, COH, SO₂NH₂, S(O)ₘA, -(CH₂)ₙ-COOR^{2'} oder -O-(CH₂)ₒ-COOR^{2'} substituiertes Phenyl, Naphthyl oder Biphenyl,
m, n jeweils unabhängig voneinander 0, 1 oder 2,
o 1, 2 oder 3,
T ein- oder zweifach durch =NR², =N-CN, =S oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl, [1,3,4]-Thiadiazol-3-yl, Imidazolidin-1-yl oder 1,2-Dihydropyrazol-2-yl, die weiterhin ein- oder zweifach durch A, CONH₂ oder COOA substituiert sein können,
bedeuten;
- in lv: D fehlt oder -CH=N-CH=CH-, -CH=CH-N=CH-, -NH-N=CH-, -CH=N-NH-, -O-N=CH- oder -CH=N-O-,,
M ein Phenylring,
R¹ H, CH₂NH₂, CONH₂, C(=S)NH₂ oder NH₂,
R^{1'} H,
W unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazoldiyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y unsubstituiertes oder ein- oder zweifach durch A, Br, Cl oder F substituiertes Phenylen,
A unverzweigtes oder verzweigtes Alkyl mit 1, 2, 3, 4, 5 oder 6 C-Atomen und/oder auch 1-7 H-Atome durch F ersetzt sein können,
T ein- oder zweifach durch =NR², =N-CN, =S oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl, [1,3,4]-Thiadiazol-3-yl, Imidazolidin-1-yl oder 1,2-Dihydropyrazol-2-yl, die weiterhin ein- oder zweifach durch A, CONH₂ oder COOA substituiert sein können,
bedeuten;
- in lw: D fehlt oder -CH=N-CH=CH-, -CH=CH-N=CH-, -NH-N=CH-, -CH=N-NH-, -O-N=CH- oder -CH=N-O-,,
M ein Phenylring,
R¹ H, CH₂NH₂, CONH₂, C(=S)NH₂ oder NH₂,
R^{1'} H,
W unsubstituiertes oder einfach durch A substituiertes Pyrazol-diyl oder Thiazol-diyl,
X CONH,
Y unsubstituiertes oder ein- oder zweifach durch A, Br, Cl oder F substituiertes Phenylen,
T 2-Imino-pyrrolidin-1-yl, 2-Imino-piperidin-1-yl, 2-Imino-[1,3,4]thiadiazol-3-yl, 2-Imino-imidazolidin-1-yl oder 3-Imino-1,2-Dihydro-pyrazol-2-yl, sowie die entsprechenden Hydroxyimino-, Cyanimino-, Alkoxyimino- und Thioxo-Derivate, wobei die heterocyclischen Reste weiterhin ein- oder zweifach durch A, CONH₂ oder COOA substituiert sein können,
A unverzweigtes oder verzweigtes Alkyl mit 1, 2, 3, 4, 5 oder 6 C-Atomen und/oder auch 1-7 H-Atome durch F ersetzt sein können, bedeuten;
sowie ihre pharmazeutisch verwendbaren Derivate, Solvate und Stereoisomere, einschließlich deren Mischungen in allen Verhältnissen.

Weiterhin sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln laa bis lac ausgedrückt werden, die der Formel I entsprechen und worin die nicht näher bezeichneten Reste die bei der Formel I angegebene Bedeutung haben, worin jedoch
- in laa: D fehlt,
M Phenyl,
R¹ unsubstituiertes oder einfach durch OH substituiertes -C(=NH)-NH₂, 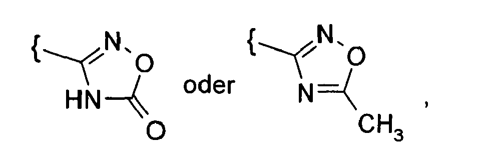 R^{1'} H,
W einen einkernigen gesättigten, ungesättigten oder aromatischen Carbo- oder Heterocyclus mit 1 bis 2 N-, O- und/oder S-Atomen, der ein- oder zweifach durch R² substituiert sein kann,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y Alkylen oder Ar-diyl,
Ar unsubstituiertes oder ein-, zwei- oder dreifach durch Hal, A, OH, NH₂, NO₂, CN, COOH, CONH₂, NHCOA, NHCONH₂, NHSO₂A, COH, SO₂NH₂, S(O)ₘA, -(CH₂)ₙ-COOR^{2'} oder -O-(CH₂)ₒ-COOR^{2'} substituiertes Phenyl, Naphthyl oder Biphenyl,
m, n jeweils unabhängig voneinander 0, 1 oder 2,
o 1, 2 oder 3,
T ein- oder zweifach durch =NR², =S oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl oder 1,2-Dihydropyrazol-2-yl, die weiterhin ein- oder zweifach durch A substituiert sein können, bedeuten;
- in lab: D fehlt,
M Phenyl,
R¹ unsubstituiertes oder einfach durch OH substituiertes -C(=NH)-NH₂, 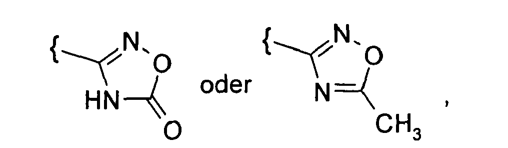 R^{1'} H,
W unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazoldiyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y unsubstituiertes oder ein- oder zweifach durch A, Br, Cl oder F substituiertes Phenylen,
A unverzweigtes oder verzweigtes Alkyl mit 1, 2, 3, 4, 5 oder 6 C-Atomen und/oder auch 1-7 H-Atome durch F ersetzt sein können,
T ein- oder zweifach durch =NR², =S oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl oder 1,2-Dihydropyrazol-2-yl, die weiterhin ein- oder zweifach durch A substituiert sein können, bedeuten;
- in lac: D fehlt,
M Phenyl, R¹ CN, NH₂, CH₂NH₂, CH₂CH₂NH₂,
unsubstituiertes oder einfach durch OH substituiertes -C(=NH)-NH₂, 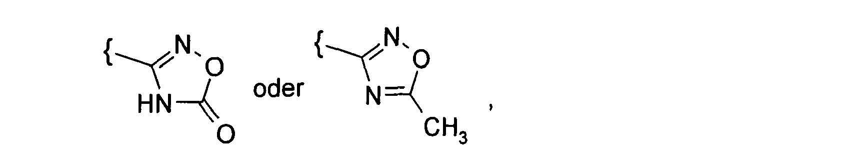 R^{1'} H,
W unsubstituiertes oder einfach durch A substituiertes Pyrazol-diyl,
X CONH,
Y unsubstituiertes oder ein- oder zweifach durch A, Br, Cl oder F substituiertes Phenylen,
T 2-Imino-pyrrolidin-1-yl, 2-Imino-piperidin-1-yl, 2-Imino-[1,3,4]thiadiazol-3-yl oder 3-Imino-1,2-Dihydro-pyrazol-2-yl, sowie die entsprechenden Hydroxyimino-, Alkoxyimino- und Thioxo-Derivate, wobei die heterocyclischen Reste weiterhin ein- oder zweifach durch A substituiert sein können,
A unverzweigtes oder verzweigtes Alkyl mit 1, 2, 3, 4, 5 oder
6 C-Atomen und/oder auch 1-7 H-Atome durch F ersetzt sein können, bedeuten;
sowie ihre pharmazeutisch verwendbaren Derivate, Solvate und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II und III sind in der Regel bekannt. Sind sie neu, so können sie aber nach an sich bekannten Methoden hergestellt werden.

Nach folgendem Schema lassen sich alle Verbindungen der folgenden Formel VI (mit R = H oder Methyl; n = 3, 4 oder 5) synthetisieren.

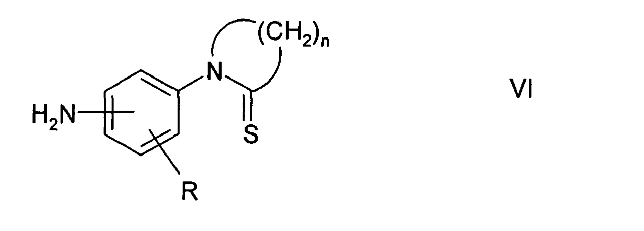

### Z.B. Synthese von 1-(4-Amino-2-methylphenyl)-piperidin-2-thion:

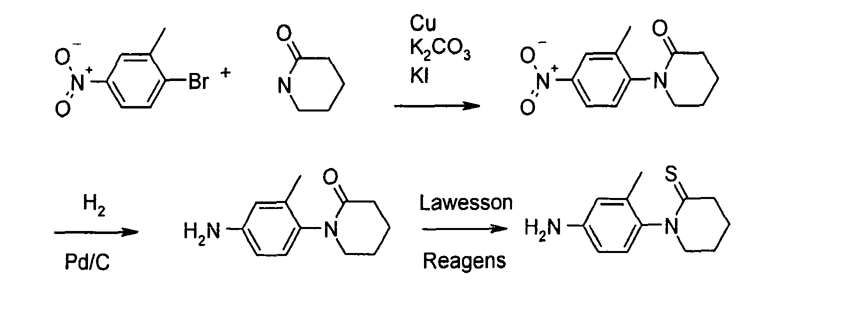

### Alternativsynthese:

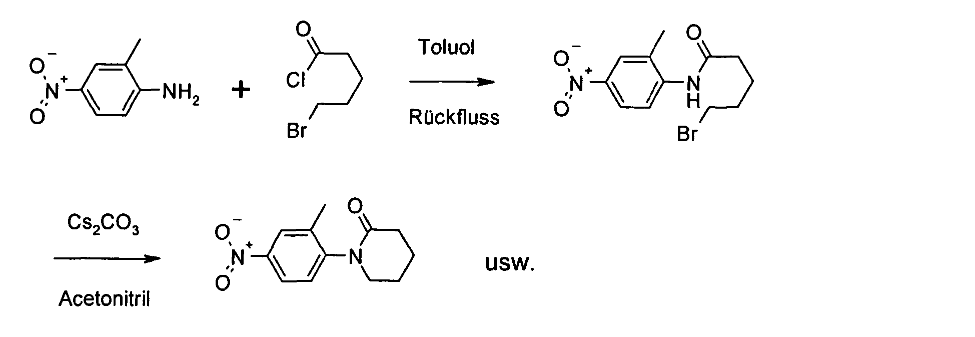

### Synthese des Phenylpiperidin-thion-bausteins ohne Methylgruppe:

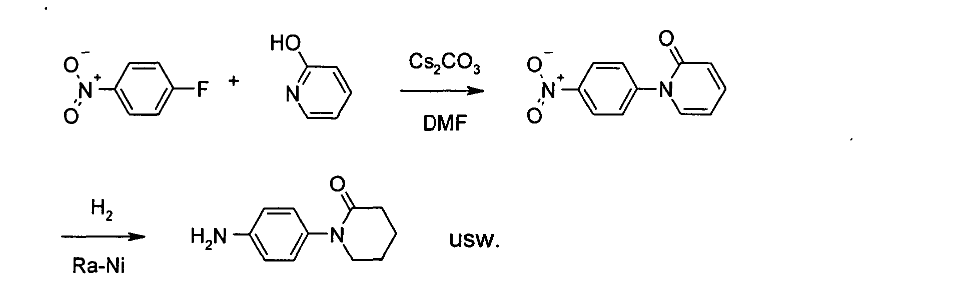

Verbindungen der Formel I können vorzugsweise erhalten werden, indem man Verbindungen der Formel II mit Verbindungen der Formel III umsetzt.

Die Umsetzung erfolgt in der Regel in einem inerten Lösungsmittel.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin, N-Methylmorpholin, oder Chinolin oder eines Überschusses der Carboxykomponente der Formel II.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Gegenstand der Erfindung ist ferner die Verwendung von Verbindungen der Formel I und/oder ihrer pharmazeutisch verwendbaren Derivate, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn nichts anderes angegeben)

### Beispiel 1

### Herstellung eines Aminbausteines:

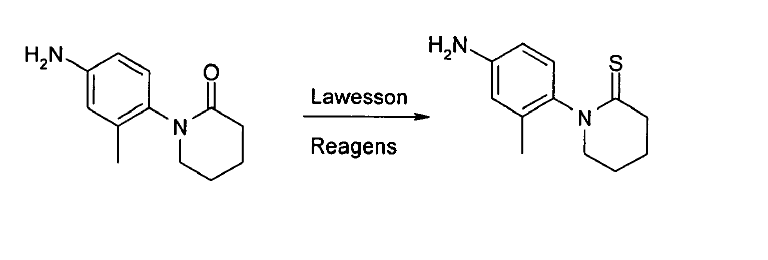

10 g (48.95 mmol) 1-(4-Amino-2-methyl-phenyl)-piperidin-2-on werden zusammen mit 9.9 g (24.48 mmol) 2,4-Bis-(4-methoxy-phenyl)-[1,3,2,4]dithiadiphosphetan 2,4-disulfide (Lawesson - Reagens) in 70ml wasserfreiem Toluol zum Sieden erhitzt. Nach 40 min. wird das Lösungsmittel entfernt und der Rückstand in Dichlormethan (DCM)/ 1 M wässriger Salzsäure aufgenommen. Nach mehrmaligem Waschen mit DCM stellt man mit konz. Natronlauge auf einen pH-Wert von 12 ein. Extraktion mit DCM, Trocknen über Na₂SO₄ und Eindampfen des Lösungsmittels liefern 9.25g (41.98 mmol) 1-(4-Amino-2-methyl-phenyl)-piperidin-2-thion.

### Beispiel 2

### Herstellung eines Aminbausteins:

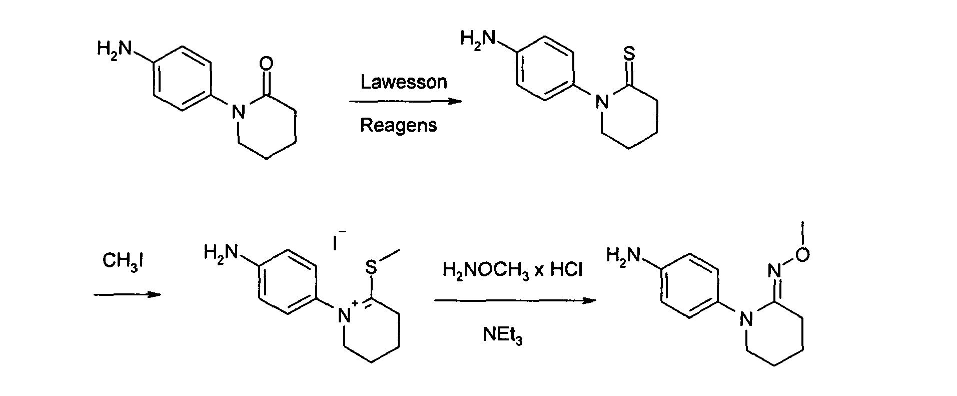

2.1 15 g (78.8 mmol) 1-(4-Aminophenyl)-piperidin-2-on werden zusammen mit 16.0 g (39.5 mmol) 2,4-Bis-(4-methoxy-phenyl)-[1,3,2,4]dithiadiphosphetan 2,4-disulfid (Lawesson-Reagens) in 100 ml wasserfreiem Toluol zum Sieden erhitzt. Nach 45 min. wird das Lösungsmittel verdampft und der Rückstand in Dichlormethan und 2 N HCl aufgenommen. Die wässrige Phase wird dreimal mit Dichlormethan extrahiert und mit konz. NaOH auf einen pH-Wert von 12 eingestellt. Extraktion mit Dichlormethan, Trocknen über Natriumsulfat und Eindampfen des Lösungsmittels liefern 1-(4-Amino-phenyl)-piperidin-2-thion als farblosen Feststoff, ESI 207.
2.2 Eine Lösung von 3.74 g (18.1 mmol) 1-(4-Amino-phenyl)-piperidin-2-thion in 30 ml Aceton wird mit 1.25 ml (20.0 mmol) lodmethan versetzt und 48 Stunden bei Raumtemperatur gerührt. Das Reaktionsgemisch wird eingedampft: 1-(4-Amino-phenyl)-6-methylsulfanyl-2,3,4,5-tetrahydropyridinium-iodid als bräunlicher Feststoff; ESI 221.
2.3 Eine Lösung von 2.68 g (12.1 mmol) 1-(4-Amino-phenyl)-6-methylsulfanyl-2,3,4,5-tetrahydropyridinium-iodid und 1.01 g (12.1 mmol) O-Methylhydroxylammoniumchlorid in 30 ml Ethanol wird mit 3.5 ml (25 mmol) Triethylamin versetzt und 20 Stunden bei Raumtemperatur gerührt. Das Reaktionsgemisch wird eingedampft, in Wasser aufgenommen und der entstandene Niederschlag abfiltriert: 1-(4-Amino-phenyl)-piperidin-2-on-O-methyl-oxim als farbloser Feststoff; ESI 220.

### Beispiel 3

### Herstellung von 2-(3-Aminomethyl-phenyl)-5-trifluormethyl-2H-pyrazol-3-carbonsäure-N-[4-(2-imino-pyrrolidin-1-yl)-phenyl]-amid ("AA")

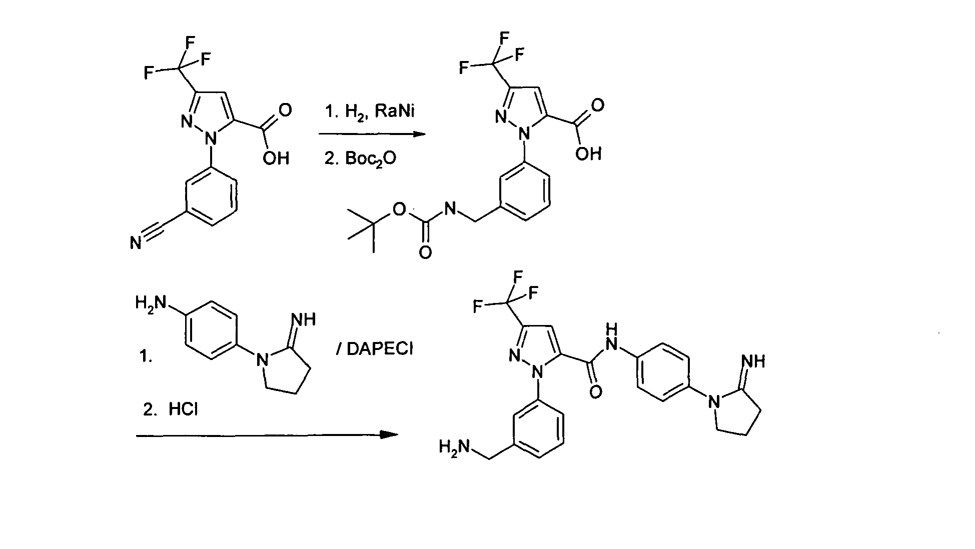

3.1 430 mg (1.53 mmol) 2-(3-Cyan-phenyl)-5-trifluoromethyl-2*H-*pyrazol-3-carbonsäure werden in 10 ml 10%igem Ammoniak in Methanol an Raney-Nickel hydriert. Nach Aufarbeitung und Fällung mit Ether erhält man 370 mg 2-(3-Aminomethyl-phenyl)-5-trifluoromethyl-2*H*-pyrazol-3-carbonsäure.
3.2 370 mg (1.3 mmol) 2-(3-Aminomethyl-phenyl)-5-trifluoromethyl-2*H-*pyrazol-3-carbonsäure werden in 6 ml Wasser suspendiert und mit 303 mg (2.86 mmol) Natriumcarbonat versetzt. Anschließend tropft man eine Lösung von 312 mg (1.43 mmol) Di-*tert*-butyldicarbonat in 12 ml 1,4-Dioxan zum Reaktionsgemisch und rührt 18 Stunden bei Raumtemperatur. Nach üblicher Aufarbeitung werden 360 mg 2-[3-(tert-Butoxycarbonylamino-methyl)-phenyl]-5-trifluoromethyl-2H-pyrazol-3-carbonsäure als Produkt isoliert.
3.3 360 mg (0.93 mmol) 2-[3-(*tert*-Butoxycarbonylamino-methyl)-phenyl]-5-trifluoromethyl-2*H*-pyrazol-3-carbonsäure werden in 2.5 ml N,N-Dimethylformamid mit 211 mg (1.0 mmol) 4-(2-Imino-pyrrolidin-1-yl)-phenylamin-hydrochlorid unter Zugabe von 192 mg (1 mmol) N-(3-Dimethylaminopropyl)-N'-ethylcarbodiimidhydrochlorid und 153 mg (1.0 mmol) 1-Hydroxybenzotriazol-hydrat umgesetzt. Nach Aufarbeitung erhält man 333 mg (3-{5-[4-(2-Imino-pyrrolidin-1-yl)-phenylcarbamoyl]-3-trifluoromethyl-pyrazol-1-yl}-benzyl)-carbaminsäure-*tert*-butyl ester.
3.4 333 mg (0.61 mmol) (3-{5-[4-(2-Imino-pyrrolidin-1-yl)-phenylcarbamoyl]-3-trifluoromethyl-pyrazol-1-yl}-benzyl)-carbaminsäure-*tert*-butyl ester werden in 1 ml Ethanol gelöst und mit 5 ml HCl in Ether versetzt. Nach Einengen und Fällen mit Ether isoliert man 289 mg 2-(3-Aminomethyl-phenyl)-5-trifluormethyl-2H-pyrazol-3-carbonsäure-N-[4-(2-imino-pyrrolidin-1-yl)-phenyl]-amid ("AA") in Form des Dihydrochlorides als Produkt.

### Beispiel 4

4.1 Analog Beispiel 3 erhält man durch Umsetzung von
   2-(3-Aminocarbonyl-phenyl)-5-trifluoromethyl-2*H*-pyrazol-3-carbonsäure mit
   1-(4-Amino-phenyl)-pyrrolidin-2-thion die Verbindung
   2-(3-Aminocarbonyl-phenyl)-5-trifluormethyl-2H-pyrazol-3-carbonsäure-N-[4-(2-thioxo-pyrrolidin-1-yl)-phenyl]-amid.
4.2 Analog Beispiel 3 erhält man durch Umsetzung von
   2-(3-Aminocarbonyl-phenyl)-5-trifluoromethyl-2*H*-pyrazol-3-carbonsäure mit
   1-(4-Amino-2-chlor-phenyl)-pyrrolidin-2-thion die Verbindung
   2-(3-Aminocarbonyl-phenyl)-5-trifluormethyl-2H-pyrazol-3-carbonsäure-N-[3-chlor-4-(2-thioxo-pyrrolidin-1-yl)-phenyl]-amid ("BB"), ESI 508.
4.3 Analog Beispiel 3 erhält man durch Umsetzung von
   2-(3-Aminocarbonyl-phenyl)-5-trifluoromethyl-2*H*-pyrazol-3-carbonsäure mit
   1-(4-Amino-phenyl)-2-imino-pyrrolidin die Verbindung
   2-(3-Aminocarbonyl-phenyl)-5-trifluormethyl-2H-pyrazol-3-carbonsäure-N-[4-(2-imino-pyrrolidin-1-yl)-phenyl]-amid, ESI 457.

### Beispiel 5

Analog Beispiel 3 erhält man durch Umsetzung von
2-(3-Aminocarbonyl-phenyl)-5-trifluoromethyl-2H-pyrazol-3-carbonsäure mit
1-(4-Amino-phenyl)-pyrrolidin-2-on-*O*-methyl-oxim,
1-(4-Amino-2-methyl-phenyl)-pyrrolidin-2-on-*O*-methyl-oxim, die Verbindungen
2-(3-Aminocarbonyl-phenyl)-5-trifluormethyl-2*H*-pyrazol-3-carbonsäure-N-[4-(2-methoxy-imino-pyrrolidin-1-yl)-phenyl]-amid, ESI 487;
2-(3-Aminocarbonyl-phenyl)-5-trifluormethyl-2*H*-pyrazol-3-carbonsäure-N-[3-methyl-4-(2-methoxy-imino-pyrrolidin-1-yl)-phenyl]-amid, ESI 515.

### Beispiel 6

6.1 Analog Beispiel 3 erhält man durch Umsetzung von 2-(3-BOC-Amino-benzo[d]isoxazol-5-yl)-5-trifluormethyl-2-H-pyrazol-3-carbonsäure mit
   4-(2-Imino-pyrrolidin-1-yl)-phenylamin,
   4-(2-Imino-5-methyl-3*H*-[1,3,4]thiadiazol-3-yl)-phenylamin,
   3-Brom-4-(2-imino-5-methyl-3*H*-[1,3,4]thiadiazol-3-yl)-phenylamin,
   4-(1,5-Dimethyl-3-imino-1,2-dihydro-pyrazol-2-yl)-phenylamin,
   und anschließender BOC-Abspaltung die Verbindungen
   2-(3-Amino-benzo[d]isoxazol-5-yl)-5-trifluormethyl-2*H*-pyrazol-3-carbonsäure-N-[4-(2-imino-pyrrolidin-1-yl)-phenyl]-amid,
   2-(3-Amino-benzo[d]isoxazol-5-yl)-5-trifluormethyl-2*H*-pyrazol-3-carbonsäure-N-[4-(2-imino-5-methyl-3*H*-[1,3,4]thiadiazol-3-yl)-phenyl]-amid,
   2-(3-Amino-benzo[d]isoxazol-5-yl)-5-trifluormethyl-2*H*-pyrazol-3-carbonsäure-N-[3-brom-4-(2-imino-5-methyl-3*H*-[1,3,4]thiadiazol-3-yl)-phenyl]-amid,
   2-(3-Amino-benzo[d]isoxazol-5-yl)-5-trifluormethyl-2*H*-pyrazol-3-carbonsäure-N-[4-(1,5-dimethyl-3-imino-1,2-dihydro-pyrazol-2-yl)-phenyl]-amid.
6.2 Analog Beispiel 3 erhält man durch Umsetzung von
   2-(3-BOC-Amino-benzo[d]isoxazol-5-yl)-5-trifluormethyl-2-H-pyrazol-3-carbonsäure mit
   1-(4-Amino-phenyl)-pyrrolidin-2-thion und anschließender BOC-Abspaltung die Verbindung
   2-(3-Amino-benzo[d]isoxazol-5-yl)-5-trifluormethyl-2*H*-pyrazol-3-carbonsäure-N-[4-(2-thioxo-pyrrolidin-1-yl)-phenyl]-amid.
6.3 Analog Beispiel 3 erhält man durch Umsetzung von
   2-(3-Aminocarbonyl-phenyl)-5-trifluoromethyl-2*H*-pyrazol-3-carbonsäure mit
   3-Brom-4-(2-imino-5-methyl-3H-[1,3,4]thiadiazol-3-yl)-phenylamin,
   4-(2-Imino-5-methyl-3*H*-[1,3,4]thiadiazol-3-yl)-phenylamin,
   4-(2-Imino-imidazolidin-1-yl)-phenylamin,
   4-(2-Imino-imidazolidin-1-yl)-3-methyl-phenylamin,
   4-(2-Cyanimino-imidazolidin-1-yl)-phenylamin,
   4-(2-Cyanimino-3-methyl-imidazolidin-1-yl)-phenylamin,
   4-(2-Imino-5-ethyl-3*H*-[1,3,4]thiadiazol-3-yl)-phenylamin,
   4-(2-Imino-5-aminocarbonyl-3*H*-[1,3,4]thiadiazol-3-yl)-phenylamin,
   4-(2-Imino-5-ethoxycarbonyl-3*H*-[1,3,4]thiadiazol-3-yl)-phenylamin,
   die Verbindungen
   2-(3-Aminocarbonyl-phenyl)-5-trifluormethyl-2*H*-pyrazol-3-carbonsäure-N-[3-brom-4-(2-imino-5-methyl-3*H*-[1,3,4]thiadiazol-3-yl)-phenyl]-amid, ESI 567, 568;
   2-(3-Aminocarbonyl-phenyl)-5-trifluormethyl-2*H*-pyrazol-3-carbonsäure-N-[4-(2-imino-5-methyl-3*H*-[1,3,4]thiadiazol-3-yl)-phenyl]-amid, ESI 488;
   2-(3-Aminocarbonyl-phenyl)-5-trifluormethyl-2*H*-pyrazol-3-carbonsäure-N-[4-(2-imino-imidazolidin-1-yl)-phenyl]-amid;
   2-(3-Aminocarbonyl-phenyl)-5-trifluormethyl-2*H*-pyrazol-3-carbonsäure-N-[4-(2-imino-imidazolidin-1-yl)-3-methyl-phenyl]-amid;
   2-(3-Aminocarbonyl-phenyl)-5-trifluormethyl-2*H*-pyrazol-3-carbonsäure-N-[4-(2-cyanimino-imidazolidin-1-yl)-phenyl]-amid ("CA");
   2-(3-Aminocarbonyl-phenyl)-5-trifluormethyl-2*H*-pyrazol-3-carbonsäure-N-[4-(2-cyanimino-3-methyl-imidazolidin-1-yl)-phenyl]-amid;
   2-(3-Aminocarbonyl-phenyl)-5-trifluormethyl-2*H*-pyrazol-3-carbonsäure-N-[4-(2-imino-5-ethyl-3*H*-[1,3,4]thiadiazol-3-yl)-phenyl]-amid ("CB"),
   2-(3-Aminocarbonyl-phenyl)-5-trifluormethyl-2*H*-pyrazol-3-carbonsäure-N-[4-(2-imino-5-aminocarbonyl-3*H*-[1,3,4]thiadiazol-3-yl)-phenyl]-amid,
   2-(3-Aminocarbonyl-phenyl)-5-trifluormethyl-2*H*-pyrazol-3-carbonsäure-N-[4-(2-imino-5-ethoxycarbonyl-3*H*-[1,3,4]thiadiazol-3-yl)-phenyl]-amid.
6.4 Analog Beispiel 3 erhält man durch Umsetzung von
   5-(3-Aminocarbonyl-phenyl)-2-methyl-thiazol-4-carbonsäure mit
   4-(2-Imino-5-ethyl-3*H*-[1,3,4]thiadiazol-3-yl)-phenylamin
   die Verbindung
   5-(3-Aminocarbonyl-phenyl)-2-methyl-thiazol-4-carbonsäure-N-[4-(2-imino-5-ethyl-3*H*-[1,3,4]thiadiazol-3-yl)-phenyl]-amid.
6.5 Analog Beispiel 3 erhält man durch Umsetzung von
   2-(3-Aminocarbonyl-phenyl)-5-methyl-2H-pyrazol-3-carbonsäure mit 4-(2-Imino-5-ethyl-3H-[1,3,4]thiadiazol-3-yl)-phenylamin die Verbindung
   2-(3-Aminocarbonyl-phenyl)-5-methyl-2*H*-pyrazol-3-carbonsäureN-[4-(2-imino-5-ethyl-3*H*-[1,3,4]thiadiazol-3-yl)-phenyl]-amid.

### Beispiel 7

7.1 Analog Beispiel 3 erhält man durch Umsetzung von
   2-(3-BOC-Amino-1 *H*-indazol-5-yl)-5-trifluormethyl-2-H-pyrazol-3-carbonsäure mit
   1-(4-Amino-phenyl)-pyrrolidin-2-on-*O*-methyl-oxim,
   1-(4-Amino-phenyl)-pyrrolidin-2-thion
   und anschließender BOC-Abspaltung die Verbindungen
   2-(3-Amino-1*H*-indazol-5-yl)-5-trifluormethyl-2*H*-pyrazol-3-carbonsäure-N-[4-(2-methoxy-imino-pyrrolidin-1-yl)-phenyl]-amid,
   2-(3-Amino-1*H*-indazol-5-yl)-5-trifluormethyl-2*H*-pyrazol-3-carbonsäure-N-[4-(2-thioxo-pyrrolidin-1-yl)-phenyl]-amid.

### Beispiel 8

Analog Beispiel 3 erhält man durch Umsetzung von
2-(3-Thiocarbamoyl-phenyl)-5-trifluoromethyl-2*H*-pyrazol-3-carbonsäure mit
1-(4-Amino-phenyl)-pyrrolidin-2-on-*O*-methyl-oxim die Verbindung
2-(3-Thiocarbamoyl-phenyl)-5-trifluormethyl-2*H*-pyrazol-3-carbonsäure-N-[4-(2-methoxy-imino-pyrrolidin-1-yl)-phenyl]-amid.

### Beispiel 9

### Herstellung von 2-(3-Aminomethyl-phenyl)-5-trifluormethyl-2H-pyrazol-3-carbonsäure-N-[4-(2-hydroxyimino-pyrrolidin-1-yl)-phenyl]-amid

Eine Lösung von 2-(3-BOC-aminomethyl-phenyl)-5-trifluormethyl-2H-pyrazol-3-carbonsäure-N-[4-(2-methylsulfanyl-pyrrolidin-1-yl)-phenyl]-amid, lodid in ketonfreiem Ethanol wird mit Hydroxylammonium Hydrochlorid und Triethylamin versetzt und bei Raumtemperatur gerührt.
Nach 20 Stunden wird zur Trockene eingedampft, der Rückstand in Wasser eingerührt und abfiltriert. Das Rohprodukt wird nach der Trocknung mit 20 ml HCl in Ether vesetzt. Nach 20 Stunden wird das Lösungsmittel im Vakuum entfernt und mit Ether ausgerührt. Man erhält 2-(3-Aminomethyl-phenyl)-5-trifluormethyl-2H-pyrazol-3-carbonsäure-N-[4-(2-hydroxyimino-pyrrolidin-1-yl)-phenyl]-amid.

### Pharmakologische Daten

### Affinität zu Rezeptoren

**Tabelle 1**

| Verbindung Nr. | FXa-IC₅₀ [M] | TF/FVIIa-IC₅₀[M] |
|---|---|---|
| "AA" | 9.6 x 10⁻⁹ | 2.3 x 10⁻⁸ |
| "BB" | 3.3 x 10⁻⁷ | 1.8 x 10⁻⁷ |
| "CA" | 2.0 x 10⁻⁷ | 6.9 x 10⁻⁸ |
| "CB" | 9.2 x 10⁻⁸ | 1.5 x 10⁻⁷ |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 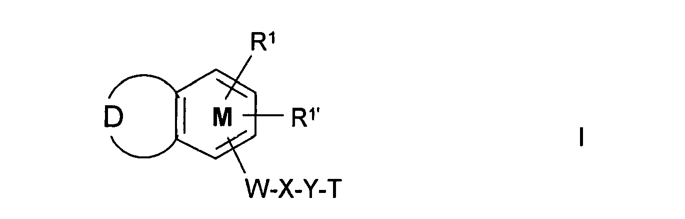 worin
D fehlt oder eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch Hal, A, -[C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²SO₂A, COR², SO₂NR² und/oder S(O)ₘA auftreten kann, und wobei ferner auch eine CH₂-Gruppe der Alkylenkette durch eine C=O-Gruppe ersetzt sein kann,
M ein Phenylring oder ein aromatischer Heterocyclus, der 1-2 N- , O- und/oder S-Atome enthalten kann,
R¹, R^{1'} jeweils unabhängig voneinander H, Hal, A, OR², N(R²)₂, NO₂, CN, COOR², CON(R²)₂, C(=S)N(R²)₂, -[C(R³)₂]ₙ-Ar, -[C(R³)₂]ₙ-Het oder -[C(R³)₂]ₙ-Cycloalkyl, -[C(R³)₂]ₙ-N(R³)₂, CN, unsubstituiertes oder einfach durch C(=O)R³, COOR³, OR³, OCOR³, OCOOR³ oder durch eine konventionelle Aminoschutzgruppe substituiertes -C(=NH)-NH₂, 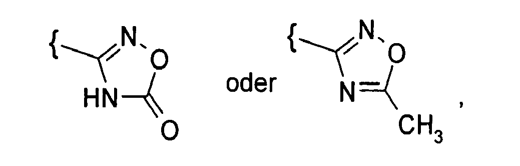
R² H, A, -[C(R³)₂]ₙ-Ar, -[C(R³)₂]ₙ-Het, -[C(R³)₂]ₙ-Cycloalkyl, -[C(R³)₂]ₙ-N(R³)₂ oder -[C(R³)₂]ₙ-OR³,
R^{2'} H, A, -[C(R³)₂]ₙ-Ar', -[C(R³)₂]ₙ-Het', -[C(R³)₂]ₙ-Cycloalkyl, -[C(R³)₂]ₙ-N(R³)₂ oder -[C(R³)₂]ₙ-OR³,
R^{2"} H, A, -[C(R³)₂]ₙ-Ar' oder -[C(R³)₂]ₙ-Cycloalkyl, -[C(R³)₂]ₙ-N(R³)₂ oder -[C(R³)₂]ₙ-OR³,
R³ H oder A,
W einen ein- oder zweikernigen gesättigten, ungesättigten oder aromatischen Carbo- oder Heterocyclus mit 1 bis 4 N-, O- und/oder S-Atomen, der ein- oder zweifach durch R² substituiert sein kann,
X CONR², CONR²C(R³)₂, -C(R³)₂NR²oder -C(R³)₂NR²C(R³)₂, -C(R³)₂O-, -C(R³)₂OC(R³)₂- oder NR²CO,
Y Alkylen, Cycloalkylen, Het-diyl oder Ar-diyl,
T einen ein- oder zweikernigen gesättigten, ungesättigten oder aromatischen Carbo- oder Heterocyclus mit 1 bis 4 N-, O- und/oder S-Atomen, der ein- oder zweifach durch =NR², =N-CN, =N-NO₂, =NOR², =NCOR², =NCOOR², =NOCOR² substituiert ist und ferner ein-, zwei- oder dreifach durch Hal, A, -[C(R³)₂]ₙ-Ar, -[C(R³)₂]ₙ-Het, -[C(R³)₂]ₙ-Cycloalkyl, OR³, N(R³)₂, NO₂, CN, COOR², CON(R²)₂, NR²COA, NR²CON(R²)₂, NR²SO₂A, COR², SO₂NR² und/oder S(O)ₘA substituiert sein kann,
A unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
Ar unsubstituiertes oder ein-, zwei- oder dreifach durch Hal, A, OR³, N(R³)₂, NO₂, CN, COOR³, CON(R³)₂, NR³COA, NR³CON(R³)₂, NR³SO₂A, COR³, SO₂N(R³)₂, S(O)ₘA, -[C(R³)₂]ₙ-COOR^{2'} oder -O-[C(R³)₂]ₒ-COOR^{2'} substituiertes Phenyl, Naphthyl oder Biphenyl,
Ar' unsubstituiertes oder ein- oder zweifach durch Hal substituiertes Phenyl oder Benzyl,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =N(R³)₂, Hal, A, -[C(R³)₂]ₙ-Ar, -[C(R³)₂]ₙ-Het¹, -[C(R³)₂]ₙ-Cycloalkyl, -[C(R³)₂]ₙ-OR^{2'}, -[C(R³)₂]ₙ-N(R^{2'})₂, NO₂, CN, -[C(R³)₂]ₙ-COOR^{2'}, -[C(R³)₂]ₙ-CON(R^{2'})₂, -[C(R³)₂]ₙ-NR^{2'}COA, NR^{2'}CON(R^{2'})₂, -[C(R³)₂]ₙ-NR^{2'}SO₂A, COR^{2'}, SO₂NR^{2'} und/oder S(O)ₘA substituiert sein kann,
Het¹ einen ein- oder zweikernigen gesättigten, ungesättigten oder aromatischen Heterocyclus mit 1 bis 2 N-, O- und/oder S-Atomen, der unsubstituiert oder ein- oder zweifach durch Carbonylsauerstoff, =S, =N(R³)₂, Hal, A, OR^{2"}, N(R^{2"})₂, NO₂, CN, COOR^{2"}, CON(R^{2"})₂, NR^{2"}COA, NR^{2"}CON(R^{2"})₂, NR^{2"}SO₂A, COR^{2"}, SO₂NR^{2"} und/oder S(O)ₘA substituiert sein kann,
Hal F, Cl, Br oder I,
n 0, 1 oder 2,
m 0, 1 oder 2,
o 1, 2 oder 3
bedeuten,
sowie ihre pharmazeutisch verwendbaren Derivate, Solvate und Stereoisomere, einschließlich deren Mischungen in allen Verhältnissen.

2. Verbindungen der Formel I nach Anspruch 1, worin
D fehlt,
sowie ihre pharmazeutisch verwendbaren Derivate, Solvate und Stereoisomere, einschließlich deren Mischungen in allen Verhältnissen.

3. Verbindungen der Formel I nach Anspruch 1 oder 2, worin
M ein Phenylring bedeutet,
sowie ihre pharmazeutisch verwendbaren Derivate, Solvate und Stereoisomere, einschließlich deren Mischungen in allen Verhältnissen.

4. Verbindungen der Formel I gemäß einem oder mehreren der Ansprüche 1-3,
worin
D eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch Hal, A, OR² oder N(R²)₂, auftreten kann, und wobei ferner auch eine CH₂-Gruppe der Alkylenkette durch eine C=O-Gruppe ersetzt sein kann,
sowie ihre pharmazeutisch verwendbaren Derivate, Solvate und Stereoisomere, einschließlich deren Mischungen in allen Verhältnissen.

5. Verbindungen der Formel I gemäß einem oder mehreren der Ansprüche 1-4,
worin
D eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch A oder NH₂, auftreten kann,
sowie ihre pharmazeutisch verwendbaren Derivate, Solvate und Stereoisomere, einschließlich deren Mischungen in allen Verhältnissen.

6. Verbindungen der Formel I gemäß einem oder mehreren der Ansprüche 1-5,
worin
D fehlt oder eine gesättigte 3- bis 4-gliedrige Alkylenkette, bei der 1 bis 3 C-Atome durch N und/oder 1 bis 2 C-Atome durch 1 bis 2 O-Atome ersetzt sein können, wobei jedoch höchstens bis zu 3 C-Atome ersetzt werden
bedeutet,
und wobei zusätzlich eine ein- oder zweifache Substitution der Alkylenkette und/oder eines darin befindlichen Stickstoffes durch NH₂ auftreten kann,
sowie ihre pharmazeutisch verwendbaren Derivate, Solvate und Stereoisomere, einschließlich deren Mischungen in allen Verhältnissen.

7. Verbindungen der Formel I gemäß einem oder mehreren der Ansprüche 1-6,
worin
D fehlt oder -CH=N-CH=CH-, -CH=CH-N=CH-, -NH-N=CH-, -CH=N-NH-, -O-N=CH- oder -CH=N-O-,
bedeutet,
und wobei zusätzlich an D eine einfache Substitution durch NH₂ auftreten kann;
sowie ihre pharmazeutisch verwendbaren Derivate, Solvate und Stereoisomere, einschließlich deren Mischungen in allen Verhältnissen.

8. Verbindungen der Formel I nach Anspruch 1
worin
R¹ H, -[C(R³)₂]ₙ-N(R³)₂, CON(R²)₂, C(=S)NH₂ oder N(R²)₂,
R^{1'} H
bedeuten,
sowie ihre pharmazeutisch verwendbaren Derivate, Solvate und Stereoisomere, einschließlich deren Mischungen in allen Verhältnissen.

9. Verbindungen der Formel I gemäß einem oder mehreren der Ansprüche 1-8,
worin
R¹ H, CH₂NH₂, CONH₂, C(=S)NH₂ oder NH₂,
R^{1'} H,
bedeuten,
sowie ihre pharmazeutisch verwendbaren Derivate, Solvate und Stereoisomere, einschließlich deren Mischungen in allen Verhältnissen.

10. Verbindungen der Formel I gemäß einem oder mehreren der Ansprüche 1-9,
worin
W einen einkernigen gesättigten, ungesättigten oder aromatischen Carbo- oder Heterocyclus mit 1 bis 2 N-, O- und/oder S-Atomen, der ein- oder zweifach durch R² substituiert sein kann,
bedeutet,
sowie ihre pharmazeutisch verwendbaren Derivate, Solvate und Stereoisomere, einschließlich deren Mischungen in allen Verhältnissen.

11. Verbindungen der Formel I gemäß einem oder mehreren der Ansprüche 1-10,
worin
W unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazoldiyl, Pyrazol-diyl, Oxazol-diyl, Isoxazol-diyl, Thiazol-diyl, Isothiazol-diyl, Pyridin-diyl, Pyrimidin-diyl, Pyrrolidin-diyl, Piperidin-diyl oder Piperazin-diyl
bedeutet,
sowie ihre pharmazeutisch verwendbaren Derivate, Solvate und Stereoisomere, einschließlich deren Mischungen in allen Verhältnissen.

12. Verbindungen der Formel I gemäß einem oder mehreren der Ansprüche 1-11,
worin
W unsubstituiertes oder einfach durch A substituiertes Pyrazoldiyl bedeutet,
sowie ihre pharmazeutisch verwendbaren Derivate, Solvate und Stereoisomere, einschließlich deren Mischungen in allen Verhältnissen.

13. Verbindungen der Formel I gemäß einem oder mehreren der Ansprüche 1-12,
worin
X CONH, CONHCH₂, CH₂NH oder CH₂NHCH₂ bedeutet,
sowie ihre pharmazeutisch verwendbaren Derivate, Solvate und Stereoisomere, einschließlich deren Mischungen in allen Verhältnissen.

14. Verbindungen der Formel I gemäß einem oder mehreren der Ansprüche 1-13,
worin
X CONH bedeutet,
sowie ihre pharmazeutisch verwendbaren Derivate, Solvate und Stereoisomere, einschließlich deren Mischungen in allen Verhältnissen.

15. Verbindungen der Formel I gemäß einem oder mehreren der Ansprüche 1-14,
worin
Y Alkylen oder Ar-diyl bedeutet,
sowie ihre pharmazeutisch verwendbaren Derivate, Solvate und Stereoisomere, einschließlich deren Mischungen in allen Verhältnissen.

16. Verbindungen der Formel I gemäß einem oder mehreren der Ansprüche 1-15,
worin
Y unsubstituiertes oder ein- oder zweifach durch A, Br, Cl oder F substituiertes Phenylen
bedeutet,
sowie ihre pharmazeutisch verwendbaren Derivate, Solvate und Stereoisomere, einschließlich deren Mischungen in allen Verhältnissen.

17. Verbindungen der Formel I gemäß einem oder mehreren der Ansprüche 1-16,
worin
T einen einkernigen gesättigten oder ungesättigten Heterocyclus mit 1 bis 3 N- , O- und/oder S- Atomen, der ein- oder zweifach durch =NR², =NOR², =N-CN, =N-NO₂, =NCOR², =NCOOR², =NOCOR² substituiert ist, sowie ein- oder zweifach durch A, CON(R²)₂ oder COOR² substituiert sein kann,
bedeutet,
sowie ihre pharmazeutisch verwendbaren Derivate, Solvate und Stereoisomere, einschließlich deren Mischungen in allen Verhältnissen.

18. Verbindungen der Formel I gemäß einem oder mehreren der Ansprüche 1-17,
worin
T einen einkernigen gesättigten oder ungesättigten Heterocyclus mit 1 bis 3 N- , O- und/oder S- Atomen, der ein- oder zweifach durch =NR², =N-CN oder =NOR² substituiert ist, sowie ein- oder zweifach durch A, CON(R²)₂ oder COOR² substituiert sein kann,
bedeutet,
sowie ihre pharmazeutisch verwendbaren Derivate, Solvate und Stereoisomere, einschließlich deren Mischungen in allen Verhältnissen.

19. Verbindungen der Formel I gemäß einem oder mehreren der Ansprüche 1-18,
worin
T ein- oder zweifach durch =NR², =N-CN, oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl, Imidazolidin-1-yl, [1,3,4]-Thiadiazol-3-yl- oder 1,2-Dihydropyrazol-2-yl, die weiterhin ein- oder zweifach durch A, CONH₂ oder COOA substituiert sein können,
bedeuten,
sowie ihre pharmazeutisch verwendbaren Derivate, Solvate und Stereoisomere, einschließlich deren Mischungen in allen Verhältnissen.

20. Verbindungen der Formel I gemäß einem oder mehreren der Ansprüche 1-19,
worin
T 2-Imino-piperidin-1-yl, 2-Imino-pyrrolidin-1-yl, 2-Imino-1*H-*pyridin-1-yl, 3-Imino-morpholin-4-yl, 4-Imino-1*H*-pyridin-1-yl, 2,6-Diimino-piperidin1-yl, 2-Imino-piperazin-1-yl, 2,6-Diiminopiperazin-1-yl, 2,5-Diimino-pyrrolidin-1-yl, 2-Imino-1,3-oxazolidin-3-yl, 3-Imino-2*H*-pyridazin-2-yl, 2-Imino-azepan-1-yl, 2-Hydroxy-6-imino-piperazin-1-yl, Pyrazol-2-yl, 1,2-Dihydropyrazol-2-yl, 2-Methoxy-6-imino-piperazin-1-yl, 2-Imino-[1,3,4]-thiadiazol-3-yl, 2-Imino-imidazolidin-1-yl sowie die entsprechenden Hydroxyimino-, Alkoxyimino- und =N-(CH₂)₁₋₃NA'₂ - Derivate, wobei A' Alkyl mit 1, 2, 3, 4, 5, oder 6 C-Atomen bedeutet, und wobei die Heterocyclen weiterhin ein- oder zweifach durch A, CONH₂ oder COOA substituiert sein können,
bedeutet,
sowie ihre pharmazeutisch verwendbaren Derivate, Solvate und Stereoisomere, einschließlich deren Mischungen in allen Verhältnissen.

21. Verbindungen der Formel I gemäß einem oder mehreren der Ansprüche 1-20,
worin
T 2-Imino-pyrrolidin-1-yl, 2-Imino-piperidin-1-yl, 2-Imino-[1,3,4]thiadiazol-3-yl, 2-Imino-imidazolidin-1-yl oder 3-Imino-1,2-Dihydro-pyrazol-2-yl, sowie die entsprechenden Hydroxyimino- und Alkoxyimino-Derivate, wobei die heterocyclischen Reste weiterhin ein- oder zweifach durch A, CONH₂ oder COOA substituiert sein können,
bedeuten,
sowie ihre pharmazeutisch verwendbaren Derivate, Solvate und Stereoisomere, einschließlich deren Mischungen in allen Verhältnissen.

22. Verbindungen der Formel I gemäß einem oder mehreren der Ansprüche 1-21,
worin
D fehlt oder -CH=N-CH=CH-, -CH=CH-N=CH-, -NH-N=CH-, -CH=N-NH-, -O-N=CH- oder -CH=N-O-,
M ein Phenylring,
R¹ H, CH₂NH₂, CONH₂, C(=S)NH₂ oder NH₂,
R^{1'} H,
W einen einkernigen gesättigten, ungesättigten oder aromatischen Carbo- oder Heterocyclus mit 1 bis 2 N-, O- und/oder S-Atomen, der ein- oder zweifach durch R² substituiert sein kann,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y Alkylen oder Ar-diyl,
Ar unsubstituiertes oder ein-, zwei- oder dreifach durch Hal, A, OH, NH₂, NO₂, CN, COOH, CONH₂, NHCOA, NHCONH₂, NHSO₂A, COH, SO₂NH₂, S(O)ₘA, -(CH₂)ₙ-COOR^{2'} oder -O-(CH₂)ₒ-COOR^{2'} substituiertes Phenyl, Naphthyl oder Biphenyl,
m, n jeweils unabhängig voneinander 0, 1 oder 2,
o 1, 2 oder 3,
T ein- oder zweifach durch =NR², =N-CN oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl, [1,3,4]-Thiadiazol-3-yl, Imidazolidin-1-yl oder 1,2-Dihydropyrazol-2-yl, die weiterhin ein- oder zweifach durch A, CONH₂ oder COOA substituiert sein können,
bedeuten,
sowie ihre pharmazeutisch verwendbaren Derivate, Solvate und Stereoisomere, einschließlich deren Mischungen in allen Verhältnissen.

23. Verbindungen der Formel I gemäß einem oder mehreren der Ansprüche 1-22,
worin
D fehlt oder -CH=N-CH=CH-, -CH=CH-N=CH-, -NH-N=CH-, -CH=N-NH-, -O-N=CH- oder -CH=N-O-,,
M ein Phenylring,
R¹ H, CH₂NH₂, CONH₂, C(=S)NH₂ oder NH₂,
R^{1'} H,
W unsubstituiertes oder ein- oder zweifach durch R² substituiertes Cyclohexan-diyl, Cyclopentan-diyl, Phenylen, Biphenylen, Furan-diyl, Thiophen-diyl, Pyrrol-diyl, Imidazoldiyl, Pyrazol-diyl, Oxazol-diyl, Isoxazol-diyi, Thiazol-diyl, Isothiazol-diyl, Pyridin-diyl, Pyrimidin-diyl, Pyrrolidin-diyl,
R² H oder Alkyl mit 1, 2, 3, 4, 5 oder 6 C-Atomen,
R^{2'} H oder Alkyl mit 1, 2, 3, 4, 5 oder 6 C-Atomen,
X CONH, CONHCH₂, CH₂NH oder CH₂NHCH₂,
Y unsubstituiertes oder ein- oder zweifach durch A, Br, Cl oder F substituiertes Phenylen,
A unverzweigtes oder verzweigtes Alkyl mit 1, 2, 3, 4, 5 oder 6 C-Atomen und/oder auch 1-7 H-Atome durch F ersetzt sein können,
T ein- oder zweifach durch =NR², =N-CN oder =NOR² substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H-*Pyridazin-2-yl, Azepan-1-yl, 2-Aza-bicyclo[2.2.2]-octan-2-yl, Pyrazol-2-yl, [1,3,4]-Thiadiazol-3-yl, Imidazolidin-1-yl oder 1,2-Dihydropyrazol-2-yl, die weiterhin ein- oder zweifach durch A, CONH₂ oder COOA substituiert sein können,
bedeuten,
sowie ihre pharmazeutisch verwendbaren Derivate, Solvate und Stereoisomere, einschließlich deren Mischungen in allen Verhältnissen.

24. Verbindungen der Formel I gemäß einem oder mehreren der Ansprüche 1-23,
worin
D fehlt oder -CH=N-CH=CH-, -CH=CH-N=CH-, -NH-N=CH-, -CH=N-NH-, -O-N=CH- oder -CH=N-O-,,
M ein Phenylring,
R¹ H, CH₂NH₂, CONH₂, C(=S)NH₂ oder NH₂,
R^{1'} H,
W unsubstituiertes oder einfach durch A substituiertes Pyrazoldiyl oder Thiazol-diyl,
X CONH,
Y unsubstituiertes oder ein- oder zweifach durch A, Br, Cl oder F substituiertes Phenylen,
T 2-Imino-pyrrolidin-1-yl, 2-Imino-piperidin-1-yl, 2-Imino-[1,3,4]thiadiazol-3-yl, 2-Imino-imidazolidin-1-yl oder 3-Imino-1,2-Dihydro-pyrazol-2-yl, sowie die entsprechenden Hydroxyimino-, Cyanimino- und Alkoxyimino-Derivate, wobei die heterocyclischen Reste weiterhin ein- oder zweifach durch A, CONH₂ oder COOA substituiert sein können,
A unverzweigtes oder verzweigtes Alkyl mit 1, 2, 3, 4, 5 oder 6 C-Atomen und/oder auch 1-7 H-Atome durch F ersetzt sein können,
bedeuten,
sowie ihre pharmazeutisch verwendbaren Derivate, Solvate und Stereoisomere, einschließlich deren Mischungen in allen Verhältnissen.

25. Verbindungen gemäß Anspruch 1 ausgewählt aus der Gruppe
2-(3-Aminomethyl-phenyl)-5-trifluormethyl-2*H*-pyrazol-3-carbonsäure-N-[4-(2-imino-pyrrolidin-1-yl)-phenyl]-amid,
2-(3-Aminocarbonyl-phenyl)-5-trifluormethyl-2*H*-pyrazol-3-carbonsäure-N-[4-(2-methoxy-imino-pyrrolidin-1-yl)-phenyl]-amid,
2-(3-Amino-benzo[d]isoxazol-5-yl)-5-trifluormethyl-2*H-*pyrazol-3-carbonsäure-N-[4-(2-imino-pyrrolidin-1-yl)-phenyl]-amid,
2-(3-Amino-benzo[d]isoxazol-5-yl)-5-trifluormethyl-2*H-*pyrazol-3-carbonsäure-N-[4-(2-imino-5-methyl-3*H*-[1,3,4]thiadiazol-3-yl)-phenyl]-amid,
2-(3-Amino-benzo[d]isoxazol-5-yl)-5-trifluormethyl-2*H-*pyrazol-3-carbonsäure-N-[4-(1,5-dimethyl-3-imino-1,2-dihydropyrazol-2-yl)-phenyl]-amid,
2-(3-Amino-1*H*-indazol-5-yl)-5-trifluormethyl-2H-pyrazol-3-carbonsäure-N-[4-(2-methoxy-imino-pyrrolidin-1-yl)-phenyl]-amid,
2-(3-Thiocarbamoyl-phenyl)-5-trifluormethyl-2*H*-pyrazol-3-carbonsäure-N-[4-(2-methoxy-imino-pyrrolidin-1-yl)-phenyl]-amid,
2-(3-Aminomethyl-phenyl)-5-trifluormethyl-2H-pyrazol-3-carbonsäure-N-[4-(2-hydroxyimino-pyrrolidin-1-yl)-phenyl]-amid,
2-(3-Aminocarbonyl-phenyl)-5-trifluormethyl-2*H*-pyrazol-3-carbonsäure-N-[3-methyl-4-(2-methoxy-imino-pyrrolidin-1-yl)-phenyl]-amid,
2-(3-Aminocarbonyl-phenyl)-5-trifluormethyl-2*H*-pyrazol-3-carbonsäure-N-[4-(2-imino-pyrrolidin-1-yl)-phenyl]-amid,
2-(3-Aminocarbonyl-phenyl)-5-trifluormethyl-2*H*-pyrazol-3-carbonsäure-N-[3-brom-4-(2-imino-5-methyl-3*H*-[1,3,4]thiadiazol-3-yl)-phenyl]-amid,
2-(3-Aminocarbonyl-phenyl)-5-trifluormethyl-2*H*-pyrazol-3-carbonsäure-N-[4-(2-imino-5-methyl-3*H*-[1,3,4]thiadiazol-3-yl)-phenyl]-amid,
2-(3-Aminocarbonyl-phenyl)-5-trifluormethyl-2*H*-pyrazol-3-carbonsäure-N-[4-(2-imino-imidazolidin-1-yl)-phenyl]-amid,
2-(3-Aminocarbonyl-phenyl)-5-trifluormethyl-2*H*-pyrazol-3-carbonsäure-N-[4-(2-imino-imidazolidin-1-yl)-3-methyl-phenyl]-amid,
2-(3-Aminocarbonyl-phenyl)-5-trifluormethyl-2*H*-pyrazol-3-carbonsäure-N-[4-(2-cyanimino-imidazolidin-1-yl)-phenyl]-amid,
2-(3-Aminocarbonyl-phenyl)-5-trifluormethyl-2*H*-pyrazol-3-carbonsäure-N-[4-(2-cyanimino-3-methyl-imidazolidin-1-yl)-phenyl]-amid,
2-(3-Aminocarbonyl-phenyl)-5-trifluormethyl-2*H*-pyrazol-3-carbonsäure-N-[4-(2-imino-5-ethyl-3*H*-[1,3,4]thiadiazol-3-yl)-phenyl]-amid,
2-(3-Aminocarbonyl-phenyl)-5-trifluormethyl-2*H*-pyrazol-3-carbonsäure-N-[4-(2-imino-5-aminocarbonyl-3*H*-[1,3,4]thiadiazol-3-yl)-phenyl]-amid,
2-(3-Aminocarbonyl-phenyl)-5-trifluormethyl-2H-pyrazol-3-carbonsäure-N-[4-(2-imino-5-ethoxycarbonyl-3*H*-[1,3,4]thiadiazol-3-yl)-phenyl]-amid,
5-(3-Aminocarbonyl-phenyl)-2-methyl-thiazol-4-carbonsäureN-[4-(2-imino-5-ethyl-3*H*-[1,3,4]thiadiazol-3-yl)-phenyl]-amid,
2-(3-Aminocarbonyl-phenyl)-5-methyl-2*H*-pyrazol-3-carbonsäure-N-[4-(2-imino-5-ethyl-3*H*-[1,3,4]thiadiazol-3-yl)-phenyl]-amid,
sowie ihre pharmazeutisch verwendbaren Derivate, Solvate und Stereoisomere, einschließlich deren Mischungen in allen Verhältnissen.

26. Verfahren zur Herstellung von Verbindungen der Formel I nach den Ansprüchen 1-24 sowie ihrer pharmazeutisch verwendbaren Derivate, Solvate und Stereoisomere, **dadurch gekennzeichnet, daß** man
a) zur Herstellung einer Verbindung der Formel I,
worin X CONR² oder CONR ²C(R³)₂ bedeutet,
eine Verbindung der Formel II 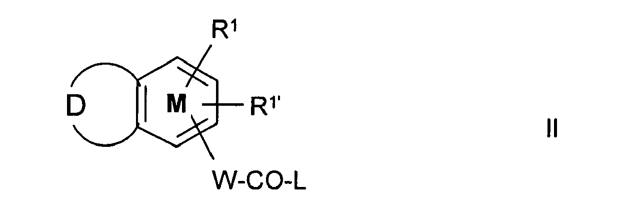 worin
L Cl, Br, I oder eine freie oder reaktionsfähig funktionell abgewandelte OH-Gruppe bedeutet bedeutet
und R¹, R^{1'}, D, M und W die in Anspruch 1 angegebenen Bedeutungen haben,
mit der Maßgabe, daß falls eine weitere OH- und/oder Aminogruppe vorliegt, diese geschützt ist,
mit einer Verbindung der Formel III
Z'-Y-T III
worin
Z' NHR² oder NHR²C(R³)₂ bedeutet
und R², Y und T die in Anspruch 1 angegebenen Bedeutungen haben,
umsetzt,
und anschließend gegebenenfalls eine Schutzgruppe abspaltet,
b) und/oder daß man in einer Verbindung der Formel I einen Rest T, R¹ und/oder R^{1'} in einen anderen Rest T, R¹ und/oder R¹ umwandelt,
indem man beispielsweise
i) eine Sulfanylverbindung in eine Iminoverbindung umwandelt,
ii) eine Aminoschutzgruppe abspaltet,
und/oder
eine Base oder Säure der Formel I in eines ihrer Salze umwandelt.

27. Verbindungen der Formel I nach einem oder mehreren der Ansprüche 1 bis 25 als Inhibitoren des Koagulationsfaktors Xa.

28. Verbindungen der Formel I nach einem oder mehreren der Ansprüche 1 bis 25 als Inhibitoren des Koagulationsfaktors Vlla.

29. Arzneimittel, enthaltend mindestens eine Verbindung der Formel I nach einem oder mehreren der Ansprüche 1 bis 25 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

30. Arzneimittel enthaltend mindestens eine Verbindung der Formel I gemäß einem oder mehreren der Ansprüche 1 bis 25 und/oder ihre pharmazeutisch verwendbaren Derivate, Solvate und Stereoisomere, einschließlich deren Mischungen in allen Verhältnissen, und mindestens einen weiteren Arzneimittelwirkstoff.

31. Verwendung von Verbindungen gemäß einem oder mehreren der Ansprüche 1 bis 25 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

32. Set (Kit), bestehend aus getrennten Packungen von
(a) einer wirksamen Menge an einer Verbindung der Formel I gemäß einem oder mehreren der Ansprüche 1 bis 25 und/oder ihrer pharmazeutisch verwendbaren Derivate, Solvate und Stereoisomere, einschließlich deren Mischungen in allen Verhältnissen,
und
(b) einer wirksamen Menge eines weiteren Arzneimittelswirkstoffs.

33. Verwendung von Verbindungen der Formel I gemäß einem oder mehreren der Ansprüche 1 bis 25 und/oder ihrer pharmazeutisch verwendbaren Derivate, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.
